# EUROPEAN PATENT APPLICATION

(11) **EP 4 717 193 A1**
(43) Date of publication of application: **01.04.2026**
(21) Application number: 24842392.3
(22) Date of filing: 17.07.2024
(51) Int. Cl.: A61B 17/115

(54) **ELECTRIC ANASTOMAT**

(30) Priority: 18.07.2023 CN 202310883083
(71) Applicant: B. J. Zh. F. Panther Medical Equipment Co., Ltd., Beijing 102209 (CN)
(72) Inventor: LIU, Qing, Beijing 102209 (CN); CHEN, Xiaoqiang, Beijing 102209 (CN); YANG, Xuelan, Beijing 102209 (CN); LIU, Libo, Beijing 102209 (CN)
(74) Representative: Clarke, Modet y Cía., S.L.
(86) International application number: PCT/CN2024/105965
(87) International publication number: WO 2025/016403

(57) **Abstract**

An electric anastomat, which comprises an anastomosis assembly and a driving assembly, wherein the driving assembly comprises: a main driving assembly, a rotating assembly, and an angular movement assembly; the main driving assembly is used for driving the anastomosis assembly to close, anastomose, or retract; the rotating assembly is used for driving the anastomosis assembly to rotate 360 degrees; and the angular movement assembly is used for driving the anastomosis assembly to move left and right; the main driving assembly, the rotating assembly, and the angular movement assembly each comprise a motor and a gear assembly, and the main driving assembly, the rotating assembly, and the swing assembly are mutually unaffected by each other during operation; the electric anastomat further comprises a main control chip, an angular movement positioning assembly, and a mount confirmation assembly, wherein the main control chip is cooperates with the angular movement positioning assembly to confirm whether the angular movement assembly is at the zero position, and the main control chip cooperates with the mount confirmation assembly to confirm whether the anastomosis assembly has been correctly mounted. By the provision of an electric assembly, the electric anastomat is more intelligent, and consequently surgical efficiency can be improved, and the difficulty of manipulation by a doctor can be reduced.

## Description

### CROSS-REFERENCE TO RELATED APPLICATION

The present application claims priority to Chinese Patent Application No. 202310883083.2, filed on July 18, 2023, the disclosure of which is hereby incorporated by reference in its entirety.

### TECHNICAL FIELD

The present application relates to the field of medical devices, and more particularly to an electric stapler.

### BACKGROUND

Endoscopic staplers are commonly used instruments in surgery, and can help doctors quickly complete the action of cutting and suturing tissue. With the development of technology, electric endoscopic staplers have been widely used, and a part of operations of the staplers adopts an electric form instead of a manual form, which enables doctors to realize the part of the operations of the endoscopic staplers by touching buttons on a handle with their fingers. However, the movement of the stapler includes various actions such as swing and rotation of an actuator and closing and firing of a nail cartridge, and also needs to adopt various electric components for intelligent control, and the intelligence degree of the electric staplers in the related art is not enough. Therefore, how to realize the intelligence of various operations to make the electric staplers more convenient to operate is a technical problem that needs to be solved urgently.

### SUMMARY

In view of this, the present application aims to solve the technical problem of how to realize more intelligent operation of an electric stapler.

Embodiments of the present application provide an electric stapler, including an anastomosis assembly and a driving assembly. The driving assembly includes a main driving assembly, a rotation assembly, and a swing angle assembly. The main driving assembly is configured to drive the anastomosis assembly to perform closure, anastomosis, and retraction, the rotation assembly is configured to drive the anastomosis assembly to rotate 360 degrees, and the swing angle assembly is configured to drive the anastomosis assembly to swing left and right.

Each of the main driving assembly, the rotation assembly and the swing angle assembly includes a motor and a gear assembly, and the main driving assembly, the rotation assembly, and the swing angle assembly do not affect each other during operation.

The electric stapler further includes a main control chip, a swing angle positioning assembly and a mounting confirmation assembly. The main control chip and the swing angle positioning assembly cooperate with each other to confirm whether the swing angle assembly is in a zero position. The main control chip and the mounting confirmation assembly cooperate with each other to confirm whether the anastomosis assembly is correctly mounted.

Further, the swing angle positioning assembly includes a swing angle positioning switch and a positioning blocking component, and the swing angle positioning switch detects the positioning blocking component to determine a position of the swing angle assembly; and/or the mounting confirmation assembly includes a mounting switch and a mounting blocking component, and the mounting switch detects the mounting blocking component to determine whether the anastomosis assembly is correctly mounted.

Further, the electric stapler includes a firing push rod, a firing rod sleeve and a mounting slider. One end of the firing push rod is connected to the main driving assembly. When the anastomosis assembly is installed, another end of the firing push rod is connected to the anastomosis assembly, and the firing push rod passes through the rotation assembly and the swing angle assembly. The mounting slider is arranged to sheathe the firing push rod, an end face of the mounting slider abuts against an end face of the firing rod sleeve arranged to sheathe the firing push rod, each of the mounting slider and the firing rod sleeve is slidable along an axis of the firing push rod, and another end face of the firing rod sleeve abuts against the mounted anastomosis assembly. After the anastomosis assembly is mounted, the anastomosis assembly pushes the firing rod sleeve to displace, to in turn push the mounting slider to displace towards a proximal end.

Further, the main driving assembly includes a firing motor, a firing gear, a firing driven gear, and a firing rack, a distal end of the firing rack is connected to a proximal end of the firing push rod, and the firing push rod is freely rotatable about the axis relative to the firing rack. The firing gear meshes with the firing driven gear, and the firing driven gear meshes with the firing rack. When the firing motor rotates in a forward direction or in a reverse direction according to a main control instruction, a rotational movement is converted into a rectilinear movement through the firing gear, the firing driven gear and the firing rack, to drive the anastomosis assembly to perform closure, anastomosis, and retraction.

Further, the electric stapler includes a barrel assembly, the anastomosis assembly is mounted on the barrel assembly, the rotation assembly includes a rotation motor, a rotation driving gear and a rotation driven gear, and the rotation assembly is connected to the barrel assembly through at least one fixing block. The rotation driving gear meshes with the rotation driven gear.

Further, the rotation driven gear includes a gear part and a cylinder part, the gear part includes a central circular hole, and an end of the cylinder part passes through the central circular hole and is fixed to the gear part.

Further, the fixing block is provided with a first snapping boss and a second snapping boss, the cylinder part of the rotation driven gear is provided with a cylinder part snapping groove corresponding to the second snapping boss, and the barrel assembly is provided with a barrel assembly snapping groove. The fixing block is fixed to the rotation driven gear through the second snapping boss and the cylinder part snapping groove, and the fixing block is fixed to the barrel assembly through the first snapping boss and the barrel assembly snapping groove.

Further, there are two fixing blocks, the two fixing blocks are snap-fitted to each other by a snapping assembly, and the two fixing blocks after being snap-fitted to each other surround an outer periphery of the cylinder part.

Further, the snapping assembly includes a convex pillar provided on one of the two fixing blocks, and a groove provided on another one of the two fixing blocks and provided corresponding to the convex pillar.

Further, the swing angle assembly includes a swing angle motor, a swing angle lead screw, a swing angle driving gear and a swing angle driven gear. The swing angle driving gear meshes with the swing angle driven gear. The swing angle driven gear helically cooperates with the swing angle lead screw. The swing angle motor rotates and drives the swing angle lead screw to move forward and backward through the swing angle driving gear and the swing angle driven gear. The swing angle lead screw passes through the rotation driven gear and is movable forward and backward. The swing angle lead screw is a hollow structure, and the firing push rod passes through the hollow structure of the swing angle lead screw and is movable forward and backward in the swing angle lead screw. The firing rod sleeve is arranged to sheathe the firing push rod, passes through the hollow structure of the swing angle lead screw, and is movable forward and backward in the swing angle lead screw.

Further, the electric stapler further includes a main circuit board and a sub circuit board, one of the main circuit board and the sub circuit board is located at one side of the electric stapler, another one of the main circuit board and the sub circuit board is located at another side of the electric stapler. A control circuit and a first guide button of the electric stapler are arranged on the main circuit board, and a second guide button is arranged on the sub circuit board. The first guide button and the second guide button are symmetrically arranged and have a same function.

Further, each of the first guide button and the second guide button has trigger functions in five directions including left and right, forward and backward, and downward pressing, respectively corresponding to a rotation of the anastomosis assembly, a left and right swing of the anastomosis assembly, and start firing confirmation of the anastomosis assembly.

Further, the positioning blocking component is two lead screw travel pieces, the two lead screw travel pieces are arranged side by side on the swing angle lead screw, a gap is arranged between the two lead screw travel pieces, and a width of the gap is greater than or equal to an induction width of the swing angle positioning switch. When the swing angle lead screw moves forward and backward, the swing angle positioning switch detects the gap. When the swing angle positioning switch detects that the gap is located at a middle position of the swing angle positioning switch, the swing angle positioning switch feeds back information to the main control chip, and the main control chip sends a signal to prompt that the swing angle assembly is in the zero position.

Further, the mounting confirmation assembly includes a mounting switch and a mounting blocking component, and the mounting switch detects the mounting blocking component to determine whether the anastomosis assembly is correctly mounted. The mounting blocking component is a slider flange arranged on the mounting slider. After the anastomosis assembly is mounted, the firing rod sleeve pushes the mounting slider to displace, the slider flange arranged on the mounting slider touches or blocks the mounting switch, potential of the mounting switch is changed, potential change information is transmitted to the main control chip, and the main control chip sends a signal to prompt that the anastomosis assembly has been mounted.

Further, after the anastomosis assembly is mounted and before the anastomosis assembly is closed, a swing angle and a rotation angle of the anastomosis assembly are adjustable by the first guide button or the second guide button. After the anastomosis assembly is closed, the main control chip locks swing and rotation functions, and the swing angle and the rotation angle of the anastomosis assembly are unadjustable. If the swing angle and the rotation angle of the anastomosis assembly need to be adjusted again, the electric stapler needs to be fired and resumed to an initial position of the electric stapler.

With such a design, the present application has at least the following advantages:
(1) The electric stapler of the present application is provided with the electric main driving assembly, the rotation assembly and the swing angle assembly, so that the doctor can conveniently operate the electric stapler, which can improve the efficiency and shorten the surgery time.
(2) The electric stapler of the present application is provided with the swing angle positioning assembly and the mounting confirmation assembly, which can improve the accuracy of the electric stapler and reduce the operation difficulty.
(3) The electric stapler of the present application adopts buttons to operate, which can realize single-handed operation, and greatly reduce the operation difficulty.
(4) The present application has an ingenious structure, a good effect, a low cost and easy process realization.

### BRIEF DESCRIPTION OF THE DRAWINGS

FIG. 1 is a schematic diagram of the overall structure of an electric stapler according to the present application.
FIG. 2 is a schematic diagram of an internal structure of a driving part of an electric stapler according to the present application.
FIG. 3 is a schematic diagram of a swing angle assembly and a rotation assembly according to the present application.
FIG. 4 is a schematic diagram of an internal structure of a swing angle assembly and a rotation assembly according to the present application.
FIG. 5 is a schematic diagram of a mounting structure for a first circuit board according to the present application.
FIG. 6 is a schematic diagram of a mounting structure for a second circuit board according to the present application.
FIG. 7 is a schematic diagram of a swing angle positioning assembly and a mounting confirmation assembly according to the present application.
FIG. 8 is a schematic diagram of a fixing block according to the present application.
FIG. 9 is a schematic diagram of a swing angle lead screw according to the present application.
FIG. 10 is a schematic diagram of a swing angle crank according to the present application.
FIG. 11 is a schematic diagram illustrating a zero position state of a swing angle according to the present application.
FIG. 12 is a schematic diagram illustrating a first limit state of a swing angle according to the present application.
FIG. 13 is a schematic diagram illustrating a second limit state of a swing angle according to the present application.
FIG. 14 is a schematic diagram illustrating a cooperation of a firing push rod and a swing angle lead screw according to the present application.

### DETAILED DESCRIPTION

It should be noted that embodiments and features in the embodiments of the present application may be combined with each other without conflict, and the detailed description should be understood as an explanation of the present application and should not be regarded as an undue limitation of the present application.

In the description of the present application, orientation or positional relationships indicated by the terms "upper", "lower", and the like are based on orientation or positional relationships shown in the accompanying drawings and are merely for convenience of describing the present application and simplification of the description, and do not indicate or imply that the referred device or element must have a particular orientation and must be constructed and operated in a particular orientation, and therefore cannot be construed as a limitation of the embodiments of the present application.

An end of each component close to the operator is defined as a proximal end, and an end of each component away from the operator is defined as a distal end. The expression of the proximal end and the distal end is for simplicity and clarity of the description only, and should not be construed as a limitation of the present application in any way.

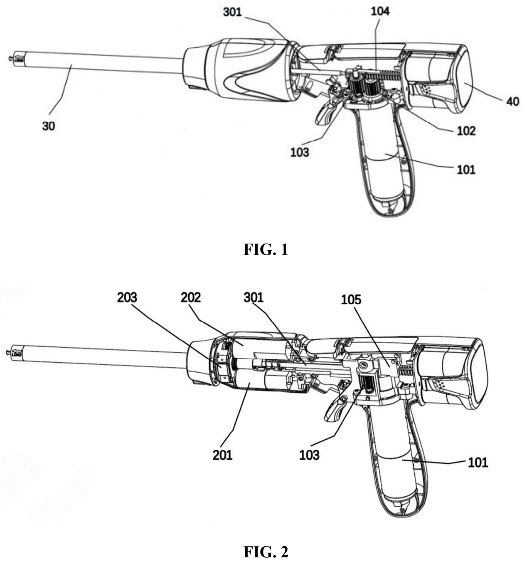

Referring to FIG. 1 to FIG. 13, an electric stapler includes an anastomosis assembly and a driving assembly. The driving assembly includes a main driving assembly, a rotation assembly, and a swing angle assembly. The main driving assembly is configured to drive the anastomosis assembly to perform closure, anastomosis, and retraction. The rotation assembly is configured to drive the anastomosis assembly to rotate 360 degrees. The swing angle assembly is configured to drive the anastomosis assembly to swing left and right. Each of the main driving assembly, the rotation assembly and the swing angle assembly includes a motor and a gear assembly. The electric stapler further includes a main control chip, a swing angle positioning assembly and a mounting confirmation assembly. The main control chip and the swing angle positioning assembly cooperate with each other to confirm whether the swing angle assembly is in a zero position, and the main control chip and the mounting confirmation assembly cooperate with each other to confirm whether the anastomosis assembly is correctly mounted. The main driving assembly, the rotation assembly, and the swing angle assembly do not affect each other during the operation.

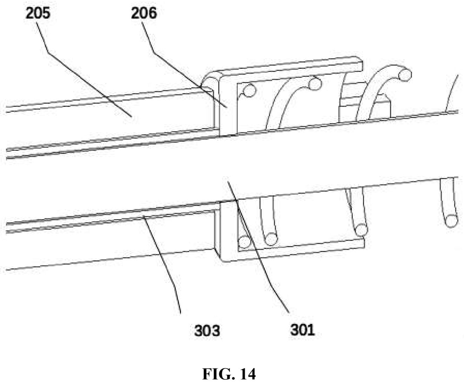

Further, referring to FIG. 14, the electric stapler includes a firing push rod 301, one end of the firing push rod 301 is connected to the main driving assembly, and when the anastomosis assembly is installed, another end of the firing push rod 301 is connected to the anastomosis assembly, and the firing push rod 301 passes through the rotation assembly and the swing angle assembly. The electric stapler further includes a firing rod sleeve 303 and a mounting slider 206, the mounting slider 206 is arranged to sheathe the firing push rod 301, and an end face of the mounting slider 206 abuts against an end face of the firing rod sleeve 303 also arranged to sheathe the firing push rod 301. Each of the mounting slider 206 and the firing rod sleeve 303 is slidable along an axis of the firing push rod 301. The firing rod sleeve 303 passes through a central through hole of a swing angle lead screw 205, and another end face of the firing rod sleeve 303 abuts against the mounted anastomosis assembly. After the anastomosis assembly is mounted, the anastomosis assembly pushes the firing rod sleeve 303 to displace, to in turn push the mounting slider 206 to displace towards the proximal end.

The main driving assembly includes a firing motor 101, a firing gear 102, a firing driven gear 103, and a firing rack 104. A distal end of the firing rack 104 is connected to a proximal end of the firing push rod 301, and the firing push rod 301 is freely rotatable about the axis relative to the firing rack 104. The firing gear 102 meshes with the firing driven gear 103, and the firing driven gear 103 meshes with the firing rack 104. When the firing motor 101 rotates in a forward direction or in a reverse direction according to a main control instruction, the rotational movement is converted into a rectilinear movement through the firing gear 102, the firing driven gear 103 and the firing rack 104, to drive the anastomosis assembly to perform closure, anastomosis, and retraction.

Referring to FIG. 2 to FIG. 4, the rotation assembly includes a rotation motor 201, a rotation driving gear 2032, and a rotation driven gear 2031, and the rotation assembly is connected to a barrel assembly 30 through a fixing block 2041. The rotation driven gear 2031 includes a gear part and a cylinder part, the gear part includes a central circular hole, and an end of the cylinder part passes through the central circular hole and is fixed to the gear part. The rotation driving gear 2032 meshes with the rotation driven gear 2031, the rotation motor 201 includes a non-circular output shaft, the rotation driving gear 2032 includes a central hole with a shape matching with the shape of the output shaft. The output shaft passes through the central hole of the rotation driving gear 2032, and drives the rotation driving gear 2032 to rotate around an axis thereof. The rotation of the rotation driving gear 2032 drives the rotation driven gear 2031 to rotate, and drives the barrel assembly 30 to rotate through the fixing block 2041.

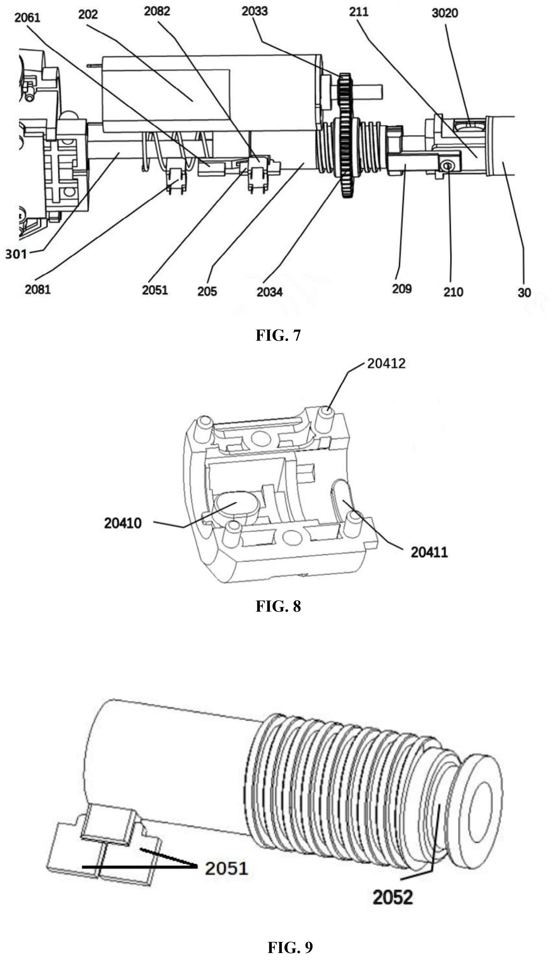

Further, there are two fixing blocks 2041, and the two fixing blocks 2041 are snap-fitted to each other by a snapping assembly. The specific manner of the structure of the snapping assembly is not limited, as long as it is ensured that the two fixing blocks 2041 can be snap-fitted to each other. Preferably, referring to FIG. 8, the snapping assembly includes a convex pillar 20412 provided on one of the two fixing blocks 2041, and a groove provided on another one of the two fixing blocks 2041 and provided corresponding to the convex pillar 20412. Each fixing block 2041 is provided with a first snapping boss 20410 and a second snapping boss 20411. The cylinder part of the rotation driven gear 2031 is provided with a cylinder part snapping groove 20310 corresponding to the second snapping boss 20411. The barrel assembly 30 is provided with a barrel assembly snapping groove 3020. The fixing block 2041 is fixed to the rotation driven gear 2031 through the second snapping boss 20411 and the cylinder part snapping groove 20310. The fixing block 2041 is fixed to the barrel assembly 30 through the first snapping boss 20410 and the barrel assembly snapping groove 3020. When the rotation driven gear 2031 rotates, the rotation driven gear 2031 drives the barrel assembly 30 and the anastomosis assembly mounted on the barrel assembly 30 to rotate through the fixing block 2041, thereby realizing the rotation function of the anastomosis assembly arranged at the distal end.

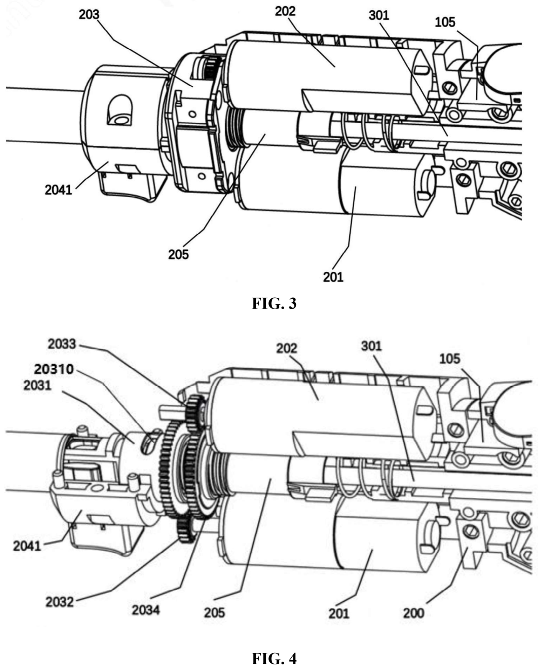

Referring to FIG. 3 and FIG. 4, the swing angle assembly includes a swing angle motor 203, a swing angle lead screw 205, a swing angle driving gear 2033 and a swing angle driven gear 2034. The swing angle driving gear 2033 meshes with the swing angle driven gear 2034, and the swing angle driven gear 2034 helically cooperates with the swing angle lead screw 205. The swing angle motor 203 rotates and drives the swing angle lead screw 205 to move forward and backward through the swing angle driving gear 2033 and the swing angle driven gear 2034. The swing angle lead screw 205 passes through the cylinder part of the rotation driven gear 2031, and is movable forward and backward in the cylinder part. The swing angle lead screw 205 is a hollow structure, and the firing push rod 301 passes through the swing angle lead screw 205 and is movable forward and backward in the swing angle lead screw 205.

Further, the swing angle driven gear 2034 is provided with a central hole, a wall of the central hole is provided with threads, and an outer wall of the swing angle lead screw 205 is correspondingly provided with threads. Preferably, the threads may be triangular threads, trapezoidal threads or rectangular threads. The swing angle motor 203 includes a non-circular output shaft, the swing angle driving gear 2033 is provided with a central hole with a shape matching with the shape of the output shaft. The output shaft passes through the central hole of the swing angle driving gear 2033, and drives the swing angle driving gear 2033 to rotate around an axis thereof. The rotation of the swing angle driving gear 2033 drives the swing angle driven gear 2034 to rotate, and the rotation of the swing angle driven gear 2034 drives the swing angle lead screw 205 to move forward and backward.

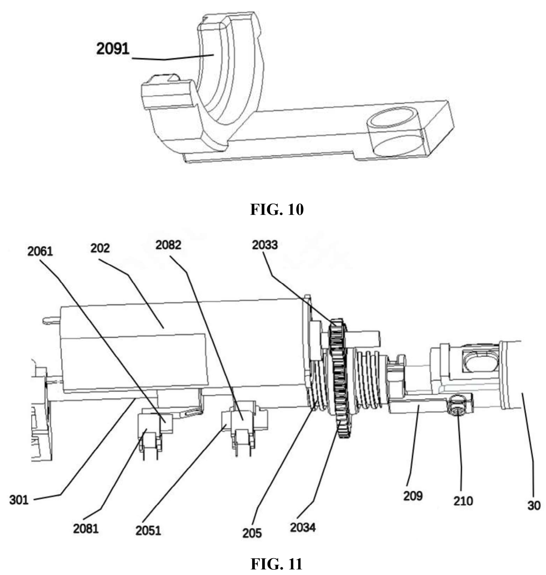

Further, referring to FIG. 7, the swing angle assembly further includes a swing angle crank 209 and a swing angle connecting rod 211. The swing angle lead screw 205 is provided with a crank annular groove 2052 (referring to FIG. 9), and the crank annular groove 2052 cooperates with an annular snapping boss 2091 arranged on the swing angle crank 209 (referring to FIG. 10). The swing angle crank 209 is fixedly connected to the swing angle connecting rod 211 through a crank pull pin 210. When the swing angle lead screw 205 moves forward and backward, the swing angle lead screw 205 drives the swing angle crank 209 to move forward and backward, to in turn drive the swing angle connecting rod 211 to move forward and backward, thereby realizing the left and right swing function of the anastomosis assembly arranged at the distal end.

Referring to FIG. 2 and FIG. 4, the electric stapler further includes a first support 105, a second support 200, and a gear carrier 203. The second support 200 and the gear carrier 203 are fixed on the first support 105, and the main driving assembly is fixed on the first support 105. The rotation motor 201 and the swing angle motor 202 are fixed on the second support 200, and the gear assemblies of the rotation assembly and the swing angle assembly are arranged in the gear carrier 203.

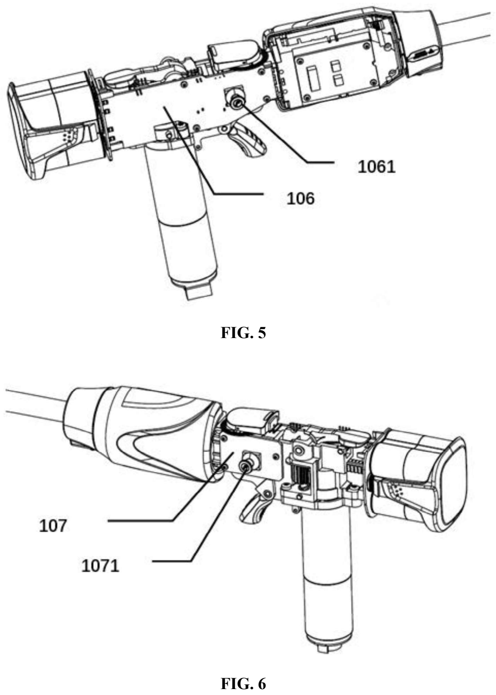

Further, referring to FIG. 5 to FIG. 6, the electric stapler includes a main circuit board 106 and a sub circuit board 107. One of the main circuit board 106 and the sub circuit board 107 is fixed on one side of the first support 105, and another one of the main circuit board 106 and the sub circuit board 107 is fixed on another side of the first support 105. A control circuit and a first guide button 1061 of the electric stapler are arranged on the main circuit board 106, and a second guide button 1071 is arranged on the sub circuit board 107. The first guide button 1061 and the second guide button 1071 are symmetrically arranged and have the same function. Each of the first guide button 1061 and the second guide button 1071 has trigger functions in five directions, that is, left and right, forward and backward, and downward pressing, respectively corresponding to the rotation of the anastomosis assembly, the left and right swing of the anastomosis assembly, and the start firing confirmation of the anastomosis assembly.

Further, referring to FIG. 7 and FIG. 9, the swing angle positioning assembly includes a swing angle positioning switch 2082 and a positioning blocking component, and the swing angle positioning switch 2082 detects the positioning blocking component to determine the position of the swing angle assembly.

Preferably, the positioning blocking component is a lead screw travel piece 2051. There may be two lead screw travel pieces 2051, the two lead screw travel pieces 2051 are arranged side by side on the swing angle lead screw 205, and a gap is arranged between the two lead screw travel pieces 2051, and the width of the gap is greater than or equal to the induction width of the swing angle positioning switch 2082. When the swing angle lead screw 205 moves forward and backward, the swing angle positioning switch 2082 detects the gap. When the swing angle positioning switch 2082 detects that the gap is located at a middle position of the swing angle positioning switch 2082, the swing angle positioning switch 2082 feeds back information to the main control chip, and the main control chip sends a signal to prompt that the swing angle assembly is in a zero position.

The mounting confirmation assembly includes a mounting switch 2081 and a mounting blocking component, and the mounting switch 2081 detects the mounting blocking component to determine whether the anastomosis assembly is correctly mounted. The mounting blocking component is a slider flange 2061 arranged on the mounting slider 206. After the anastomosis assembly is mounted, the firing rod sleeve 303 pushes the mounting slider 206 to displace, the slider flange 2061 arranged on the mounting slider 206 touches or blocks the mounting switch 2081, the potential of the mounting switch 2081 is changed, potential change information is transmitted to the main control chip, and the main control chip sends a signal to prompt that the anastomosis assembly has been mounted.

Further, the electric stapler further includes a swing angle driving electric plate, the mounting switch 2081 and the swing angle positioning switch 2082 are fixed on the swing angle driving electric plate, and the swing angle driving electric plate is arranged on the first support 105.

Referring to FIG. 1, the electric stapler further includes a removable battery assembly 40 for powering the electric stapler. The main driving assembly is provided with a firing zero position confirmation assembly. After the battery assembly 40 is mounted, the main control chip detects whether the battery assembly 40 satisfies a use condition. If the use condition is not satisfied, the main control chip sends out an alarm signal. If the use condition is satisfied, the main control chip sends an instruction to the firing motor 101 to identify a firing zero position through the firing zero position confirmation assembly, and a next operation is performed after the firing zero position is identified successfully.

The specific structure of the firing zero position confirmation assembly is not limited, as long as the firing zero position can be confirmed.

After the firing zero position is identified successfully, the main control chip sends an instruction to the swing angle motor 202 to identify a swing angle zero position. The swing angle motor 202 starts to rotate, so that the swing angle driving gear 2033 drives the swing angle driven gear 2034 to rotate, to in turn drive the swing angle lead screw 205 to move forward and backward, and the swing angle positioning switch 2082 detects the gap between the lead screw travel pieces 2051 arranged on the swing angle lead screw 205. After the swing angle positioning switch 2082 detects the gap, the swing angle positioning switch 2082 feeds back information to the main control chip to determine the swing angle zero position. After the swing angle zero position is successfully identified, the main control chip sends a signal to the operator, and a disposable anastomosis assembly is further mounted by the operator. After the anastomosis assembly is mounted, the anastomosis assembly pushes the firing rod sleeve 303 to displace toward the proximal end, the firing rod sleeve 303 passes through the central through hole of the swing angle lead screw 205 to push the mounting slider 206 to displace toward the proximal end, and the slider flange 2061 arranged on the mounting slider 206 touches or blocks the mounting switch 2081 to allow the potential of the mounting switch 2081 to be changed, thereby providing a signal to the main control chip that the anastomosis assembly has been mounted.

After the anastomosis assembly is successfully mounted, the main control chip waits for the anastomosis assembly information to be flashed in. At this time, the operator needs to flash an electronic tag storing the anastomosis assembly information in an instrument identification area, and the main control chip reads and writes the electronic tag information, and calls the corresponding logic program to perform anastomosis control. At this time, the instrument is fully activated, and the jaw (i.e., the anastomosis assembly) can be closed and opened, the jaw (i.e., the anastomosis assembly) can be rotated, and the swing angle of the jaw (i.e., the anastomosis assembly) can be adjusted. When necessary, the operator can trigger the four directions of the guide button (the first guide button 1061 or the second guide button 1071) respectively to adjust the swing angle or rotation of the jaw (i.e., the anastomosis assembly).

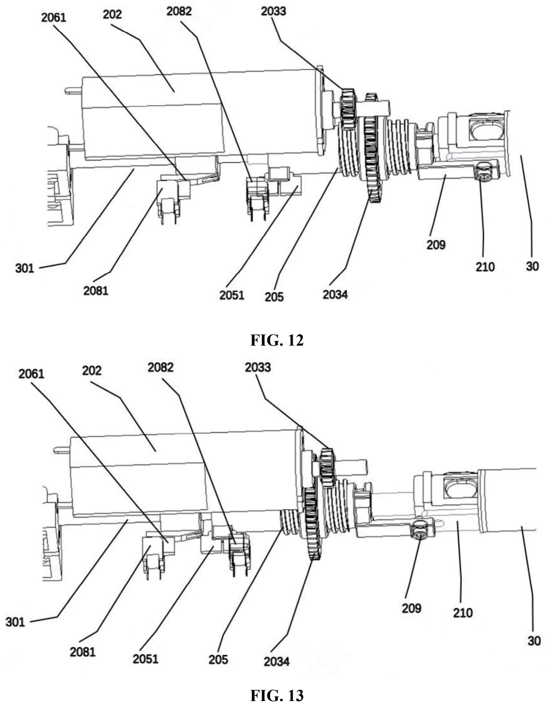

When the guide button (the first guide button 1061 or the second guide button 1071) is pressed forward and backward, the swing angle motor 202 rotates, the swing angle lead screw 205 is driven to move forward and backward through the swing angle driving gear 2033 and the swing angle driven gear 2034, and the swing angle crank 209 and the swing angle connecting rod 211 are displaced with the displacement of the swing angle lead screw 205, thereby realizing the swing angle of the anastomosis assembly. When the gap between the lead screw travel pieces 2051 is aligned with the center of the swing angle positioning switch 2082 (referring to FIG. 10, corresponding to the swing angle zero position) or when a left side or right side of the lead screw travel pieces 2051 is aligned with the center of the swing angle positioning switch 2082 (referring to FIG. 11 and FIG. 12, corresponding to a first limit state of the swing angle and a second limit state of the swing angle), the system is automatically stopped. The system can be stopped by releasing the guide button (the first guide button 1061 or the second guide button 1071) at other positions.

When the guide button (the first guide button 1061 or the second guide button 1071) is pressed left and right, the rotation motor 201 runs, and the rotation driving gear 2032 drives the rotation driven gear 2031 to rotate. Since the barrel assembly 30 is fixed on the rotation driven gear 2031 through the fixing block 2041, the barrel assembly 30 rotates together with the rotation of the rotation driven gear 2031, to realize the rotation function of the barrel assembly 30 and the nail cartridge assembly (i.e., the anastomosis assembly). The rotation can be stopped by releasing the guide button (the first guide button 1061 or the second guide button 1071).

After the anastomosis assembly is mounted and before it is closed, the angle and orientation thereof are adjusted by the guide button (the first guide button 1061 or the second guide button 1071) according to the surgery needs. The center position of the guide button (the first guide button 1061 or the second guide button 1071) is pressed to start the firing confirmation function, but this function can be used normally only when the anastomosis assembly is closed in place. Each time the guide button (the first guide button 1061 or the second guide button 1071) is triggered in one direction by the operator, the on/off signal is transmitted to the main chip, and after the signal is identified by the main chip, the main chip controls the corresponding logic to cooperate with the execution component to realize the function. After the anastomosis assembly is closed, the main control chip locks the swing and rotation functions, and the swing angle and the rotation angle of the anastomosis assembly can no longer be adjusted. If the swing angle and the rotation angle of the anastomosis assembly need to be adjusted again, the electric stapler needs to be fired and resumed to the initial position of the electric stapler.

In the description of the present application, an expression with reference to the terms "an embodiment", "some embodiments", "an example", "a specific example", "some examples" or the like means that specific features, structures, materials, or characteristics described in combination with the embodiment or example are included in at least one embodiment or example of the embodiments of the present application. In the present application, the schematic expression of the above terms is not necessarily directed to the same embodiment or example. Moreover, the specific features, structures, materials, or characteristics described may be combined with each other in any one or more embodiments or examples in a suitable manner. Furthermore, those skilled in the art can combine different embodiments or examples described in the present application and features of different embodiments or examples without contradicting each other.

The foregoing is merely a preferred embodiment of the present application, and is not intended to limit the present application, and various modifications and variations can be made for those skilled in the art. Any modifications, substitutions and improvements made within the spirit and principles of the present application should be included within the scope of protection of the present application.

## Claims

1. An electric stapler, comprising an anastomosis assembly and a driving assembly, wherein the driving assembly comprises a main driving assembly, a rotation assembly, and a swing angle assembly; the main driving assembly is configured to drive the anastomosis assembly to perform closure, anastomosis, and retraction, the rotation assembly is configured to drive the anastomosis assembly to rotate 360 degrees, and the swing angle assembly is configured to drive the anastomosis assembly to swing left and right;
each of the main driving assembly, the rotation assembly and the swing angle assembly comprises a motor and a gear assembly, and the main driving assembly, the rotation assembly, and the swing angle assembly do not affect each other during operation;
the electric stapler further comprises a main control chip, a swing angle positioning assembly and a mounting confirmation assembly, the main control chip and the swing angle positioning assembly cooperate with each other to confirm whether the swing angle assembly is in a zero position, and the main control chip and the mounting confirmation assembly cooperate with each other to confirm whether the anastomosis assembly is correctly mounted.

2. The electric stapler according to claim 1, wherein the swing angle positioning assembly comprises a swing angle positioning switch and a positioning blocking component, and the swing angle positioning switch detects the positioning blocking component to determine a position of the swing angle assembly; and/or the mounting confirmation assembly comprises a mounting switch and a mounting blocking component, and the mounting switch detects the mounting blocking component to determine whether the anastomosis assembly is correctly mounted.

3. The electric stapler according to claim 1 or 2, wherein the electric stapler comprises a firing push rod, a firing rod sleeve and a mounting slider, one end of the firing push rod is connected to the main driving assembly, wherein when the anastomosis assembly is installed, another end of the firing push rod is connected to the anastomosis assembly, and the firing push rod passes through the rotation assembly and the swing angle assembly; wherein the mounting slider is arranged to sheathe the firing push rod, an end face of the mounting slider abuts against an end face of the firing rod sleeve arranged to sheathe the firing push rod, each of the mounting slider and the firing rod sleeve is slidable along an axis of the firing push rod, and another end face of the firing rod sleeve abuts against the mounted anastomosis assembly, wherein after the anastomosis assembly is mounted, the anastomosis assembly pushes the firing rod sleeve to displace, to in turn push the mounting slider to displace towards a proximal end.

4. The electric stapler according to claim 3, wherein the main driving assembly comprises a firing motor, a firing gear, a firing driven gear, and a firing rack, a distal end of the firing rack is connected to a proximal end of the firing push rod, and the firing push rod is freely rotatable about the axis relative to the firing rack; the firing gear meshes with the firing driven gear, and the firing driven gear meshes with the firing rack; wherein when the firing motor rotates in a forward direction or in a reverse direction according to a main control instruction, a rotational movement is converted into a rectilinear movement through the firing gear, the firing driven gear and the firing rack, to drive the anastomosis assembly to perform closure, anastomosis, and retraction.

5. The electric stapler according to any one of claims 1 to 4, wherein the electric stapler comprises a barrel assembly, the anastomosis assembly is mounted on the barrel assembly, the rotation assembly comprises a rotation motor, a rotation driving gear and a rotation driven gear, and the rotation assembly is connected to the barrel assembly through at least one fixing block, wherein the rotation driving gear meshes with the rotation driven gear.

6. The electric stapler according to claim 5, wherein the rotation driven gear comprises a gear part and a cylinder part, the gear part comprises a central circular hole, and an end of the cylinder part passes through the central circular hole and is fixed to the gear part.

7. The electric stapler according to claim 6, wherein the fixing block is provided with a first snapping boss and a second snapping boss, the cylinder part of the rotation driven gear is provided with a cylinder part snapping groove corresponding to the second snapping boss, the barrel assembly is provided with a barrel assembly snapping groove, the fixing block is fixed to the rotation driven gear through the second snapping boss and the cylinder part snapping groove, and the fixing block is fixed to the barrel assembly through the first snapping boss and the barrel assembly snapping groove.

8. The electric stapler according to claim 6, wherein there are two fixing blocks, the two fixing blocks are snap-fitted to each other by a snapping assembly, and the two fixing blocks after being snap-fitted to each other surround an outer periphery of the cylinder part.

9. The electric stapler according to claim 8, wherein the snapping assembly comprises a convex pillar provided on one of the two fixing blocks, and a groove provided on another one of the two fixing blocks and provided corresponding to the convex pillar.

10. The electric stapler according to claim 3 or 4, wherein the swing angle assembly comprises a swing angle motor, a swing angle lead screw, a swing angle driving gear and a swing angle driven gear, the swing angle driving gear meshes with the swing angle driven gear, the swing angle driven gear helically cooperates with the swing angle lead screw, the swing angle motor rotates and drives the swing angle lead screw to move forward and backward through the swing angle driving gear and the swing angle driven gear; the swing angle lead screw passes through the rotation driven gear and is movable forward and backward; the swing angle lead screw is a hollow structure, and the firing push rod passes through the swing angle lead screw and is movable forward and backward in the swing angle lead screw.

11. The electric stapler according to any one of claims 1 to 10, wherein the electric stapler further comprises a main circuit board and a sub circuit board, one of the main circuit board and the sub circuit board is located at one side of the electric stapler, another one of the main circuit board and the sub circuit board is located at another side of the electric stapler, a control circuit and a first guide button of the electric stapler are arranged on the main circuit board, and a second guide button is arranged on the sub circuit board, wherein the first guide button and the second guide button are symmetrically arranged and have a same function.

12. The electric stapler according to claim 11, wherein each of the first guide button and the second guide button has trigger functions in five directions comprising left and right, forward and backward, and downward pressing, respectively corresponding to a rotation of the anastomosis assembly, a left and right swing of the anastomosis assembly, and start firing confirmation of the anastomosis assembly.

13. The electric stapler according to claim 10, wherein the positioning blocking component is two lead screw travel pieces, the two lead screw travel pieces are arranged side by side on the swing angle lead screw, a gap is arranged between the two lead screw travel pieces, and a width of the gap is greater than or equal to an induction width of the swing angle positioning switch; wherein when the swing angle lead screw moves forward and backward, the swing angle positioning switch detects the gap, wherein when the swing angle positioning switch detects that the gap is located at a middle position of the swing angle positioning switch, the swing angle positioning switch feeds back information to the main control chip, and the main control chip sends a signal to prompt that the swing angle assembly is in the zero position.

14. The electric stapler according to claim 3, wherein the mounting confirmation assembly comprises a mounting switch and a mounting blocking component, and the mounting switch detects the mounting blocking component to determine whether the anastomosis assembly is correctly mounted; wherein the mounting blocking component is a slider flange arranged on the mounting slider, wherein after the anastomosis assembly is mounted, the firing rod sleeve pushes the mounting slider to displace, the slider flange arranged on the mounting slider touches or blocks the mounting switch, potential of the mounting switch is changed, potential change information is transmitted to the main control chip, and the main control chip sends a signal to prompt that the anastomosis assembly has been mounted.

15. The electric stapler according to claim 11, wherein after the anastomosis assembly is mounted and before the anastomosis assembly is closed, a swing angle and a rotation angle of the anastomosis assembly are adjustable by the first guide button or the second guide button; wherein after the anastomosis assembly is closed, the main control chip locks swing and rotation functions, and the swing angle and the rotation angle of the anastomosis assembly are unadjustable, wherein if the swing angle and the rotation angle of the anastomosis assembly need to be adjusted again, the electric stapler needs to be fired and resumed to an initial position of the electric stapler.
